# FASCICULE DE BREVET EUROPEEN

(11) **EP 1 648 569 B1**
(45) Date de publication et mention de la délivrance du brevet: **06.12.2006**
(21) Numéro de dépôt: 04767366.0
(22) Date de dépôt: 17.06.2004
(51) Int. Cl.: A61Q 19/06, A61Q 19/08, A61K 8/49

(54) **UTILISATION COSMETIQUE D'UNE COMPOSITION COMPRENANT AU MOINS UNE OXAZOLINE, A TITRE DE PRINCIPE ACTIF, COMME AMINCISSANT ET/OU POUR PREVENIR ET/OU TRAITER LA CELLULITE**
KOSMETISCHE VERWENDUNG EINER ZUSAMMENSETZUNG MIT MINDESTENS EINEM OXAZOLIN ALS WIRKSTOFF, ALS ABNEHMPRODUKT UND/ODER ZUR PRÄVENTION UND/ODER BEHANDLUNG VON CELLULITE
COSMETIC USE OF A COMPOSITION CONTAINING AT LEAST ONE OXAZOLINE, SERVING AS AN ACTIVE SUBSTANCE, AS A SLIMMING PRODUCT AND/OR FOR PREVENTING AND/OR TREATING CELLULITE

(30) Priorité: 18.06.2003 FR 0307333
(43) Date de publication de la demande: 26.04.2006
(73) Titulaire: Laboratoires Expanscience, 92400 Courbevoie (FR)
(72) Inventeur: MSIKA, Philippe, F-78000 Versailles (FR); PICCIRILLI, Antoine, F-78000 Versailles (FR); PICCARDI, Nathalie, F-38120 Saint Egreve (FR)
(74) Mandataire: Warcoin, Jacques
(86) Numéro de dépôt international: PCT/FR2004/001504
(87) Numéro de publication internationale: WO 2004/112741

(56) Documents cités:
- US-A- 4 876 249
- PATENT ABSTRACTS OF JAPAN vol. 2003, no. 03, 5 mai 2003 (2003-05-05) & JP 2002 338555 A (ONO PHARMACEUT CO LTD), 27 novembre 2002 (2002-11-27)
- PATENT ABSTRACTS OF JAPAN vol. 1998, no. 05, 30 avril 1998 (1998-04-30) & JP 10 017565 A (SANKYO CO LTD), 20 janvier 1998 (1998-01-20)

## Description

La présente invention se rapporte à l'utilisation d'une composition cosmétique à action amincissante, comprenant, à titre de principe actif, au moins une oxazoline. La présente invention se rapporte également à l'utilisation d'une telle composition pour prévenir et/ou traiter la cellulite.

L'amincissement dans le cadre de la présente invention passe préférentiellement par la lutte contre la surcharge pondérale localisée.

L'adiposité, ou excès de graisse dans le tissu cellulaire sous-cutané, peut avoir de nombreuses causes plus ou moins complexes, plus ou moins connues ou plus ou moins comprises.

Le tissu adipeux, variété particulière de tissu conjonctif, est constitué d'adipocytes, séparés par des cloisons, elles-mêmes délimitées par des travées conjonctivovasculaires. La partie conjonctive comporte des fibres de collagènes, de réticuline et des cellules réticulo-endothéliales.

Les adipocytes contiennent des quantités variables de graisses sous la forme de triglycérides, ces triglycérides étant synthétisés in vivo par les adipocytes eux-mêmes, selon des réactions de type enzymatique à partir des acides gras libres et du glycérol, produit de dégradation du glucose, contenus dans l'organisme et apportés à celui-ci par l'alimentation. Or, parallèlement, les triglycérides ainsi formés, puis stockés, dans les cellules adipocytes peuvent également se redécomposer, toujours sous l'action d'enzymes spécifiques contenues dans ces mêmes cellules, en libérant cette fois des acides gras d'une part et du glycérol et/ou des mono- et/ou des di-esters du glycérol d'autre part. Les acides gras ainsi relargués peuvent alors soit diffuser dans l'organisme pour y être consommés ou transformés de différentes façons, soit être recaptés, aussitôt ou un peu plus tard, par les adipocytes pour générer à nouveau des triglycérides.

Les adipocytes emmagasinent donc les calories d'origine alimentaire sous forme de triglycérides puis les transforment en acides gras libres en faisant appel à des systèmes enzymatiques. Les adipocytes, qui jouent ainsi un rôle essentiel dans la synthèse des lipides, leur stockage et leur libération dans le sang, sont régulées par la lipogénèse, qui correspond à la formation de triglycérides par réaction enzymatique entre des acides gras et le glycérol provenant du glucose, et la lipolyse, qui correspond à la décomposition enzymatique de triglycérides en acides gras et glycérol.

Ces transformations se réalisent notamment sous le contrôle de médiateurs tels que l'adrénaline, les oestrogènes, qui vont bloquer ou non les lipides au sein des adipocytes, les récepteurs alpha, qui bloquent la lipolyse, et les récepteurs bêta, qui facilitent la lipolyse.

La cellulite, ou lipodystrophie localisée, est caractérisée par une infiltration oedémateuse du tissu adipeux qui altère l'esthétique et l'harmonie de la silhouette.

Si, pour des raisons diverses, telles qu'une nourriture trop riche, l'inactivité et/ou le vieillissement, un déséquilibre substantiel s'installe dans l'organisme entre la lipogenèse et la lipolyse, c'est à dire plus précisément si les quantités de graisses formées par lipogenèse deviennent notablement et constamment supérieures à celles qui sont éliminées par lipolyse, il se produit alors dans les adipocytes une accumulation de triglycérides, qui, si elle devient excessive, peut se traduire par une surcharge pondérale localisée et/ou progressivement par l'apparition d'une peau épaisse, à surface souvent irrégulière, d'aspect dit "peau d'orange", et de consistance plus ou moins flasque ou gélatineuse, donnant finalement à la silhouette un aspect général disgracieux.

Ce tissu cellulitique n'épargne pas les hommes mais est nettement plus fréquent chez les femmes, qu'elles soient minces ou rondes. Les masses graisseuses se localisent préférentiellement sur la moitié inférieure du corps, au niveau des hanches, des cuisses, du ventre, sans oublier les genoux et les chevilles.

La cellulite résulte notamment d'un stockage des triglycérides dans les adipocytes, qui peuvent augmenter de volume de façon considérable, en effet, ces cellules peuvent atteindre, selon les circonstances, 40 µm à 120 µm de diamètre, soit une augmentation de 27 fois en volume, et d'une augmentation de la viscosité de la substance fondamentale du derme, qui se traduit par une rétention d'eau et une diminution des échanges cellulaires. Ces deux mécanismes entraînent une compression des vaisseaux sanguins et lymphatiques et une congestion des tissus.

Pour prévenir la cellulite, il faut notamment :
- diminuer la formation de triglycérides; c'est-à-dire diminuer la lipogenèse ; et/ou
- augmenter la lipolyse ; et/ou
- restaurer une microcirculation active et régulière ; et/ou
- limiter les oedèmes.

Compte tenu du profond inconfort tant physique qu'esthétique, et parfois psychologique, qu'elles occasionnent auprès des individus qui en sont atteints, en particulier chez les femmes, la surcharge pondérale localisée et la cellulite constituent de nos jours des affections de moins en moins bien supportées ou acceptées.

Des méthodes ont déjà été proposées en vue de traiter la surcharge pondérale localisée et la cellulite, parmi celles-ci certaines, reposant sur des traitements chirurgicaux, tels que la liposuccion, permettent actuellement d'obtenir des résultats véritablement satisfaisants. Toutefois, de tels traitements présentent bien évidemment comme inconvénient majeur de nécessiter la mise en oeuvre sur le corps humain ou animal d'opérations invasives par nature délicates, non sans risques et souvent coûteuses.

Il existe également sur le marché de nombreux cosmétiques amincissants et /ou qui ont une action anti-cellulite. Comme exemples d'actifs fréquemment utilisés, on peut notamment citer les extraits végétaux, tels que les extraits de caféine, de Gingko biloba, de reine-des-prés, de Centalla asiatica, d'arnica, de noix de cola, du fracon, du lierre grimpant, de romarin, de souci, de ginseng, de millepertius, d'orthosiphon, d'algues brunes, d'algues rouges, de bouleau, qui sont des agents lipolytiques et la sphingosine et la rutine, extrait de *Ruta graveolens,* qui sont des agents liporéducteurs.

Les lipolytiques agissent au niveau de l'élimination des surcharges lipidiques (lipolyse) et les liporéducteurs luttent contre la formation de graisse (lipogenèse).

Ces actifs sont le plus souvent administrés par voie topique, mais ils peuvent aussi être administrés *per os.*

A ces actifs spécifiques, peuvent s'ajouter des actifs désinfiltrants, tels que le virbunum, la pensée sauvage, et des veinotoniques, tels que le ruscus, l'escine, qui sont souvent associés aux actifs amincissants.

Enfin, les formulations comprenant ces actifs amincissants et/ou anti-cellulite connus peuvent être complétées par des actifs restructurant et lissant qui luttent contre le relâchement de la peau.

Le domaine de la cosmétique est perpétuellement à la recherche de nouvelles molécules ou de nouveaux extraits efficaces pour lutter contre l'adiposité humaine ou animale, et ceci en vue notamment d'obtenir un effet général, ou au contraire localisé, d'amincissement et/ou d'affinement de la peau ou de la silhouette.

Dans sa demande de brevet française n° 01/16917, la Demanderesse décrit l'utilisation d'oxazolines, qui permettent l'inhibition de la migration des cellules de Langerhans.

La Demanderesse décrit ainsi un médicament, comprenant au moins une oxazoline en tant que principe actif, destiné au traitement ou à la prévention des réactions allergiques, et/ou inflammatoires, et/ou irritatives de 1a peau et/ou des muqueuses.

La demande de brevet US 4 876 249 décrit des compositions comprenant des oxazolines, dans lesquelles les oxazolines sont des promoteurs de pénétration d'agents actifs physiologiques à travers la couche *stratum corneum* de la peau.

D'une manière surprenante, la Demanderesse a découvert que les oxazolines sont également capables d'inhiber la lipogénèse dans des adipocytes humains.

La Demanderesse a ainsi découvert qu'une composition comprenant au moins une oxazoline, à titre de principe actif, peut être utilisée en tant que composition amincissante, et/ou pour prévenir et/ou traiter la cellulite.

Les oxazolines selon la présente invention répondent aux formules générales suivantes: 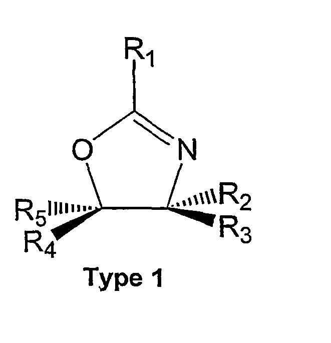 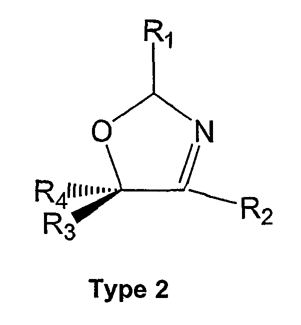 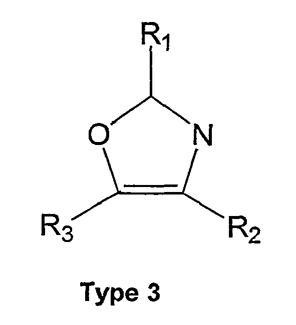 dans laquelle R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone.

Selon un mode avantageux de la présente invention, la dite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diniéthyl-2-heptadéc-8-ényl-1,3-oxazoline, de la 4-hydroxyméthyl-4-méthyl-2-heptadécyl-1,3-oxazoline, la (E)-4-hydroxyméthyl-4-méthyl-2-heptadée-8-ényl-1,3-oxazoline, la 2-undécyl-4-éthyl-4-hydroxyméthyl-1,3-oxazoline. Avantageusement, la dite oxazoline est la 2-undécyl-4,4-diméthyl-1,3-oxazoline, appelée OX100, de formule:

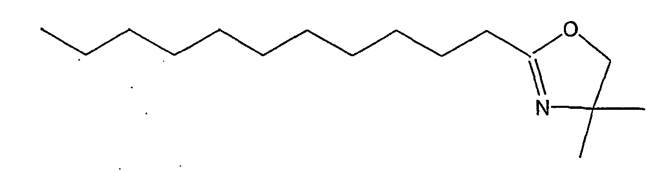

De nombreuses voies de synthèse sont connues pour préparer les composés oxazolines selon l'invention. Ainsi, celles-ci peuvent être préparées par synthèse chimique en faisant réagir un acide gras (ou un ester méthylique) et un amino-alcool, le plus souvent en présence d'un agent azéotropique afin de favoriser l'élimination de l'eau formée (et du méthanol formé). Une autre voie de synthèse possible consiste à condenser un halo-amide en présence d'une base forte ou de carbonate de sodium (R. M. Lusskin, J. Amer. Chem. Soc., 72, (1950), 5577). Les oxazolines peuvent également être synthétisées par réaction des époxydes sur les nitriles, par réaction du chlorure de thionyle sur les hydroxyamides ou encore, par action d'un acide sur une aziridinylphosphine.

Par l'expression « amincissement » ou « lutte contre la surchargé pondérale localisée », on entend selon la présente invention une action permettant d'éviter ou tout au moins de réduire la formation de graisses sous-cutanées telles que décrites précédemment. Cette action se traduit notamment par une diminution des surcharges ou réserves disgracieuses, par un affinement de la silhouette, par une accélération de l'élimination des excédents, par une meilleure définition du contour de corps ou encore une silhouette resculptée.

Par méthode de « traitement cosmétique pour lutter contre la surcharge pondérale localisée » on entend, selon la présente invention, la mise en oeuvre d'un traitement cosmétique permettant de mesurer de manière visible l'action décrite ci-dessus.

Ainsi, une composition topique comprenant une ou des oxazolines utilisée selon l'invention peut être appliquée sur les zones de la peau susceptibles de former ces surcharges pondérales localisées, à savoir des zones où ces surcharges sont déjà formées ou en cours de formation.

La composition cosmétique selon l'invention se caractérise en ce que la concentration en oxazoline est avantageusement comprise entre environ 0,01 et environ 10% en poids, et plus avantageusement entre environ 0,01 et environ 3% en poids, par rapport au poids total de la composition.

La composition qui permet la mise en oeuvre de l'invention comprend un support cosmétiquement acceptable, c'est à dire un support compatible avec la peau et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide; d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et ou non-ionique, d'un dispositif trans-dermique où sous toute autre forme pour application topique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel.

Elle peut éventuellement être appliquée sur la peau sous forme d'aérosol. Elle peut également se présenter sous forme solide, et par exemple sous forme de stick. Elle peut aussi être appliquée au moyen d'un patch.

Avantageusement, le milieu cosmétiquement acceptable est une solution huileuse, une émulsion eau-dans-huile, une émulsion huile-dans eau, une microémulsion, un gel huileux, un gel anhydre, une dispersion de vésicules, de microcapsules ou de microparticules, un dispositif trans-dermique

La composition selon l'invention peut contenir également les adjuvants habituels dans le domaine cosmétique, tels que les gélifiants hydrophiles ou lipophiles, les actifs hydrophiles ou lipophiles, les épaississants, les conservateurs, les antioxydants, les solvants, les parfums, les agents chélateurs, les absorbeurs d'odeur, des filtres chimiques ou minéraux, des pigments minéraux, les tensioactifs, les polymères, les huiles de silicone et les matières colorantes. Les quantités de ces différents adjuvants sont celles classiquement utilisées dans les domaines considérés, et par exemple de 0,01 à 20% du poids total de la composition. Ces adjuvants, selon leur nature, peuvent être introduits dans la phase grasse, dans la phase aqueuse, dans les vésicules lipidiques et ou dans les nanoparticules.

Lorsque la composition de l'invention est une émulsion, la proportion de la phase grasse peut aller de 5 à 80% en poids, et de préférence de 5 à 50% du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30% en poids, et de préférence de 0,5 à 20% du poids total de la composition.

Comme huiles utilisables dans les compositions permettant de mettre en oeuvre l'invention, on peut citer les huiles minérales, les huiles d'origine végétale (huile d'abricot, huile de tournesol, huile de prune), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stearate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyethylenes.

Les modes d'administration, les posologies et les formes galéniques optimales des composés et compositions selon l'invention peuvent être déterminés selon les critères généralement pris en compte dans l'établissement d'un traitement cosmétique, de préférence dermatologique, adapté à un patient comme par exemple le poids corporel du patient, l'excès de graisse constaté, l'aspect du tissu cellulitique, la tolérance au traitement, le type de peau.

La composition utilisée selon l'invention peut contenir d'autres actifs à action amincissante comme les lipolytiques et les liporéducteurs tels que décrits en introduction, conduisant à un effet complémentaire ou éventuellement synergique.

L'invention concerne ainsi l'utilisation d'oxazolines pour la préparation de compositions topiques utiles pour prévenir et/ou traiter la cellulite et/ou pour favoriser l'amincissement et notamment pour lutter contre la surcharge pondérale localisée, caractérisée en ce que l'on applique, de façon simultanée, séparée ou étalée dans le temps une ou plusieurs oxazolines ainsi qu'un ou plusieurs actifs amincissants de type lipolytique et/ou un ou plusieurs actifs amincissants de type liporéducteur.

L'actif amincissant de type lipolytique peut être choisi parmi : la caféine, le rhodystérol, la palmitoyl-carnitine, les bioactifs alpha et gamma, l'escine, le ginkgo biloba et la sphingosine. L'actif amincissant de type liporéducteur peut être choisi parmi : l'andiroba, la Garcinia Cambogia, la rutine.

On peut également appliquer de façon simultanée, séparée ou étalée dans le temps un ou plusieurs actifs désinfiltrants et/ou veinotoniques en plus de l'application de la composition utilisée selon l'invention. Les actifs désinfiltrants ou veinotoniques peuvent être choisis parmi : le viburnum, le lierre, l'arnica, la pisolle, la pensée sauvage, le Fucus vesiculosus, la ruscus, le ginkgo biloba et l'escine.

La composition utilisée selon l'invention peut en outre comprendre d'autres actifs tels que :
- Un extrait de fleurs sophora japonica: cet extrait est riche en flavonoïdes (anti radicalaire) et en rutine. Cet actif favorise la microcirculation, facilitant et activant le drainage et la désinfiltration des tissus ;
- L'extrait de centella asiatica : Extrait de centella, plante originaire de l'Afrique de l'est et du Madagascar. Cet actif contient des terpènes (asiaticosides, de l'acide asiatica et de l'acide madécassique), aux propriétés drainantes, désinfiltrantes et raffermissantes sur les tissus. Il est notamment utilisé dans les produits amincissants mais aussi dans les produits anti-vergetures, anti-rides et cicatrisants ; 0 à 5% d'extrait de centella peut ainsi être présent dans une composition amincissante ;
- « Hydrolyzed Soy Protein » : protéine de soja qui est un élastorégulateur. Ces peptides du soja peuvent être tout peptide obtenu par hydrolyse de protéines extraites du soja, selon des conditions opératoires connues de l'homme du métier, en d'autres termes tout hydrolysat de protéine du soja. Les peptides de soja, qui sont décrits dans la demande de brevet WO 00/19974 sont particulièrement adaptés pour être introduits dans les compositions utilisées dans le cadre de la présente invention. Cet actif permet la restauration des mécanismes de renouvellement cellulaire, active la synthèse des éléments structuraux de la matrice extracellulaire et possède une action restructurante, régénérante et raffermissante ; 0 à 5% de protéine de soja peut ainsi être présent dans une composition amincissante ;
- des actifs anti-âge et/ou raffermissant, parmi lesquels on peut citer les furanes d'avocat, le rétinol et ses dérivés, la vitamine C, la vitamine E, le silicium, ou les insaponifiables de soja;
- des agents lissants, tels que notamment l'AHA;
- des antioxydants ;
- des actifs capables de bloquer la différenciation des pré-adipocytes en adipocytes dont notamment des triterpènes, des antagonistes PPARs (*Perozysome Proliferator-Activated Receptor*), des inhibiteurs de MMPs notamment l'extrait peptidique de lupin tel que décrit dans la demande de brevet français FR 2 792 202;
- des actifs pour favoriser l'amincissement tel que les isoflavones, notamment les isoflavones décrites dans la demande de brevet français n°0207995 ;

La présente invention a également pour objet une méthode de traitement cosmétique pour favoriser l'amincissement, caractérisée en ce que l'on applique par voie topique une composition cosmétique comprenant une ou plusieurs oxazolines.

Un autre objet de la présente invention est une méthode de traitement cosmétique pour prévenir et/ou traiter la cellulite, caractérisée en ce que l'on applique par voie topique une composition comprenant une ou plusieurs oxazolines.

Un objet de la présente invention est également une méthode de traitement cosmétique pour affiner la silhouette, accélérer l'élimination des excédents, mieux définir le contour du corps et/ou resculpter la silhouette, caractérisée en ce que l'on applique par voie topique une composition comprenant une ou plusieurs oxazolines.

La surcharge pondérale se caractérise, dans le cadre de la présente invention, par un surpoids, par rapport au « poids idéal », non pathologique. Le traitement cosmétique selon l'invention permet de perdre ou d'affiner des rondeurs localisées superflues mais ne s'identifie pas à un traitement thérapeutique.

Selon une variante avantageuse de l'invention, lors de ces méthodes de traitement cosmétique, on applique par voie topique sur les zones de la peau susceptibles de former des surcharges pondérales localisées, de façon simultanée, préparée ou étalée dans le temps, une ou plusieurs oxazolines ainsi qu'un ou plusieurs actifs amincissants de type lipolytique et/ou liporéducteur, et/ou un ou des actifs désinfiltrants et/ou veinotoniques.

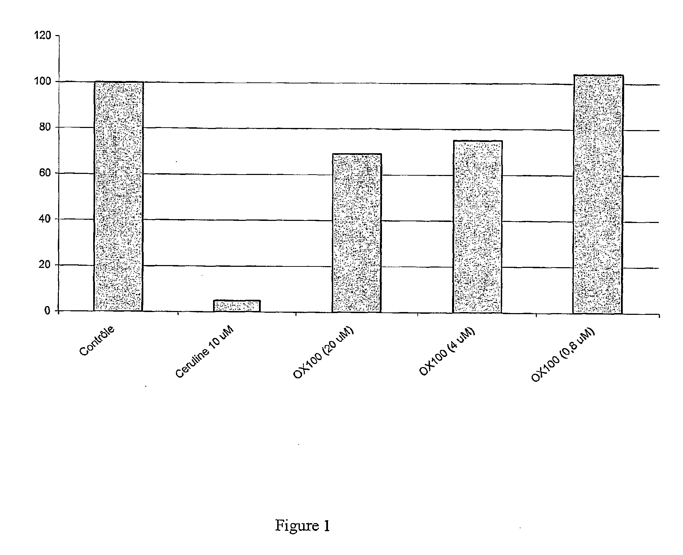

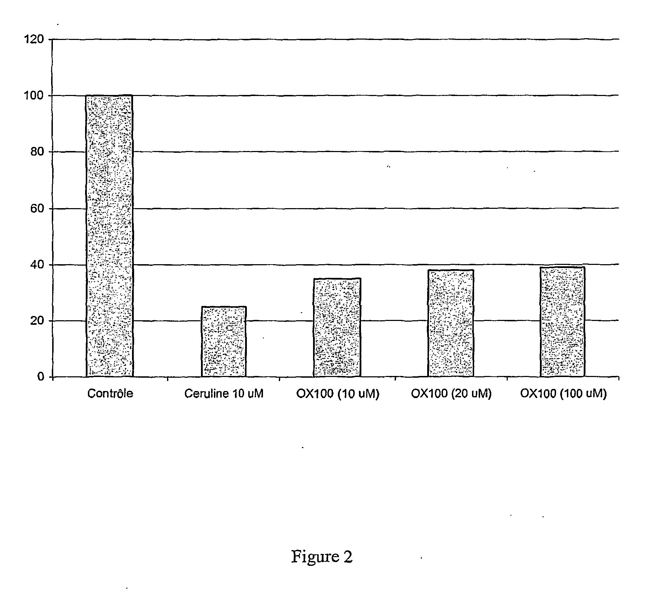

Les figures 1 et 2 illustrent l'effet de l'OX100 sur l'incorporation d'acétate radio-marqué dans les lipides adipocytaires. Les résultats sont exprimés en pourcentage du témoin et représentent l'incorporation d'acétate radiomarqué.

Les exemples suivant illustrent la présente invention.

### Exemple 1 : crème huile dans eau

| **INGREDIENTS** | **% p/p** |
|---|---|
| Eau | QSP 100 |
| Squalane | 5,00 |
| Petrolatum | 5,00 |
| Glycérine | 5,00 |
| Isodecyl Neopentanoate | 5,00 |
| Pentaerythrityl Tetraethylhexanoate | 5,00 |
| Cyclométhicone | 4,00 |
| Alcool Cétéarylique | 3,00 |
| Myristyl Myristate | 2,00 |
| Laureth-23 | 2,00 |
| Silice | 2,00 |
| Furane Heptadécadiénylique | 0,1 à 10 |
| Cire d'abeille | 1,00 |
| Gomme Sclerotium | 1,00 |
| PEG-6 | 1,00 |
| Polyacrylamide | 0,80 |
| Stéarate de Glyceryl | 0,70 |
| Diméthiconol | 0,70 |
| Glucoside de Cetearyl | 0,60 |
| C13-14 Isoparafine | 0,40 |
| Acide Citrique | 0,14 |
| Laureth-7 | 0,10 |
| 4,5,7-Trihydroxyisoflavone | 0,01 à 10 |
| Caféine | 0,1 à 10 |
| Extrait de Enteromorpha Compressa | 0,01 à 5 |
| Extrait de Garcinia Cambogia | 0,01 à 10 |
| Extrait de Ginkgo Biloba | 0,01 à 10 |
| Extrait de Fleurs de Sophora Japonica | 0,01 à 20 |
| OX100 | 0,01 à 10 |
| Conservateur | QS |
| Parfum | QS |

| | |
|---|---|
| QS = quantité suffisante QSP = quantité suffisante pour | |

### Exemple 2 : crème eau dans huile

| **INGREDIENTS** | **% p/p** |
|---|---|
| Eau | QSP 100 |
| Polyisobutène Hydrogéné | 7,00 |
| Stéarate Isocétylique | 7,00 |
| Cyclométhicone | 4,80 |
| Glycérine | 4,00 |
| Huile Minérale | 3,00 |
| Oxide de Zinc | 3,00 |
| Butylène Glycol | 2,00 |
| Isononyl Isononanoate | 2,00 |
| Cire d'abeille | 2,00 |
| Cétyl Diméthicone Copolyol | 1,70 |
| Polyglycéryl-4 Isostéarate | 1,65 |
| Hexyl laurate | 1,65 |
| Disodium tartrate | 1,60 |
| Chlorure de Sodium | 1,00 |
| PEG-6 | 1,00 |
| 4,5,7-Trihydroxyisoflavone | 0,01 à 10 |
| Retinyl palmitate | 0,01 à 10 |
| Extrait de Enteromorpha Compressa | 0,01 à 5 |
| Extrait de Fleurs de Sophora Japonica | 0,01 à 20 |
| Extrait de Centella Asiatica | 0,01 à 5 |
| OX100 | 0,01 à 10 |
| Conservateur | QS |
| Parfum | QS |

| | |
|---|---|
| QS = quantité suffisante QSP = quantité suffisante pour | |

### Exemple 3 : stick

| **INGREDIENTS** | **% p/p** |
|---|---|
| Huile de Castor | QSP 100 |
| Alcool Oléylique | 20,00 |
| Huile de Noyaux de Palme Hydrogénée | 17,00 |
| Cire Candelilla | 11,00 |
| Polyglyceryl-3 Cire d'Abeille | 10,00 |
| Huile Minérale | 9,57 |
| Heptadecadienyl Furane | 0,1 à 10 |
| Beurre de Karité | 2,00 |
| 4,5,7-Trihydroxyisoflavone | 0,01 à 10 |
| Quaternium-18 Hectorite | 1,10 |
| Dioxyde de Titane | 1,00 |
| Tocopheryl Acétate | 0,50 |
| Propylene Carbonate | 0,33 |
| Parfum | QS |
| Rétinol | 0,01 à 10 |
| Extrait de Enteromorpha Compressa | 0,01 à 5 |
| Extrait de Fleurs de Sophora Japonica | 0,01 à 20 |
| Extrait de Centella Asiatica | 0,01 à 5 |
| OX100 | 0,01 à 10 |

| | |
|---|---|
| QS = quantité suffisante QSP = quantité suffisante pour | |

### Exemple 4 : gel crème

| **INGREDIENTS** | **% p/p** |
|---|---|
| Eau | QSP 100 |
| Cyclométhicone | 5,40 |
| Octyl Palmitate | 5,00 |
| Glycérides de Coco Hydrogénés | 3,00 |
| Alcool Béhénylique d'Arachide | 2,55 |
| Propylène Glycol | 2,50 |
| Isodécyl Néopentanoate | 2,00 |
| Stéarate de Glycéryle | 1,70 |
| Alcool Cétylique | 1,30 |
| Acide Stéarique | 1,00 |
| PEG-6 | 1,00 |
| Cire d'Abeille | 0,40 |
| C13-14 Isoparafine | 0,40 |
| Butylène Glycol | 0,16 |
| Glycérine | 0,16 |
| Alcool Cétéarylique | 0,10 |
| Cetyl Palmitate | 0,10 |
| Cocoglycérides | 0,10 |
| Laureth-7 | 0,10 |
| 4,5,7-Trihydroxyisoflavone | 0,01 à 10 |
| Extrait de Enteromorpha Compressa | 0,01 à 5 |
| Extrait de Fleurs de Sophora Japonica | 0,01 à 20 |
| Extrait de Centella Asiatica | 0,01 à 5 |
| OX100 | 0,01 à 10 |
| Conservateur | QS |
| Parfum | QS |

| | |
|---|---|
| QS = quantité suffisante QSP = quantité suffisante pour | |

### Exemple 5 : spray

| **INGREDIENTS** | **% p/p** |
|---|---|
| Eau | QSP 100 |
| Glycérine | 4,00 |
| Montmorillonite | 3,00 |
| PEG-6 | 3,00 |
| Glycine | 0,30 |
| Acide Citrique | 0,09 |
| 4,5,7-Trihydroxyisoflavone | 0,01 à 10 |
| Extrait de Enteromorpha Compressa | 0,01 à 5 |
| Extrait de Fleurs de Sophora Japonica | 0,01 à 20 |
| Extrait de Centella Asiatica | 0,01 à 5 |
| OX100 | 0,01 à 10 |
| Conservateur | QS |
| Parfum | QS |

| | |
|---|---|
| QS = quantité suffisante QSP = quantité suffisante pour | |

### Exemple 6 : Evaluation de la synthèse lipidique dans des adipocytes en suspension

### Produit à l'essai

Une solution d'OX100, diluée à une concentration de 10⁻² M dans du DMSO, a été testée.

### Conditions de culture

Des adipocytes humains normaux ont été isolés à partir de biopsies abdominales (chirurgie plastique). Immédiatement après la réception, les prélèvements sont incubés pendant 30 minutes à 37°C en présence de collagénase (fournie par la société Sigma). La suspension d'adipocytes est ensuite rincée et diluée trois fois dans le milieu de culture. Le milieu de culture est constitué de :
- 1,87 mg/ml bicarbonate (fourni par la société Life Technologies),
- 25 UI/ml/ 25µg/ml de penicilline/streptomycine (fournis par la société Life Technologies),
- 2 mM de glutamine (fourni par la société Life Technologies),
- 100% v/v de MEM (fourni par la société Merck Eurolab), et
- 0,5% p/v d'albumine d'origine bovine (fourni par la société Sigma).

### Evaluation de la synthèse lipidique

Les adipocytes en suspension sont incubés pendant 1 heure à 37°C en présence de différentes concentrations d'OX100. Les concentrations en OX100 testées sont de 20 µM, 4 µM et 0,8 µM.
Un volume de 10 ml d'acétate radio-marqué (2-C¹⁴, 60,87 µCi/ml, fourni par la société Amersham) est ensuite ajouté à la préparation.
Après 4 heures d'incubation, les lipides sont extraits selon la procédure décrite par Bligh et Dyer, Can J Biochem Physiol, 37, 922, (1959), (méthanol/chloroforme/eau), évaporés sous azote et la radio-activité incorporée a été quantifiée par scintillation liquide (appareil de scintillation liquide de référence LKB 1210, fourni par la société Rackbeta).
L'éventuelle interaction entre l'acétate radio-marqué et l'OX100 a été évaluée afin de confirmer la spécificité du marquage, en incubant la plus forte concentration d'OX100 (20 µM) avec de l'acétate seul.

### Résultats

L'OX100 n'induit aucune interférence avec le radio-marquage.
Après 4 heures d'incubation, l'incorporation de C¹⁴ était élevée dans les adipocytes contrôles (185 000 cpm). Le bruit de fond à T0 était faible.
La molécule de référence, la céruline (inhibiteur de FAS, Fatty Acid Synthase) testée à 10 µM inhibe l'incorporation d'acétate (95% d'inhibition/témoin). Ce résultat valide l'essai.
L'OX100 à 20 et 4 µM diminue l'incorporation d'acétate de respectivement 31 et 25% du témoin (figure 1)
On a montré de manière tout à fait inattendue que l'OX100 est capable d'inhiber la lipogenèse dans des adipocytes humains.

### Exemple 7 : Evaluation de la synthèse lipidique dans des adipocytes en suspension

### Produit à l'essai

Une solution d'OX100 (10⁻² M dans du DMSO) a été testée.

Conditions de culture : elles sont identiques à celle de l'exemple 6

### Evaluation de la synthèse lipidique

Les adipocytes en suspension sont incubés pendant 1h à 37°C en présence de différentes concentrations d'OX100 (10, 20 et 100 µM). Un volume de 10 ml d'acétate radio-marqué (2-C¹⁴, 60,87 µCi/ml, Amersham) est ensuite ajouté à la préparation. Après 4h d'incubation, les lipides sont extraits selon la procédure décrite par Bligh et Dyer (méthanol/chloroforme/eau), évaporés sous azote et la radio-activité incorporée a été quantifiée par scintillation liquide (LKB 1210 Rackbeta).

### Résultats

L'OX100 n'induit aucune interférence avec le radio-marquage.
La molécule de référence, la céruline (inhibiteur de FAS, Fatty Acid Synthase) testée à 10 µM inhibe l'incorporation d'acétate (75% d'inhibition/témoin). Ce résultat valide l'essai.
L'OX100 testé à 10, 20 et 100 µM diminue significativement l'incorporation d'acétate dans les lipides (respectivement 35, 38 et 39% du témoin ; figure 2).

L'OX100 a montré une activité inhibitrice significative de la synthèse des lipides. L'OX100 est donc capable d'inhiber la lipogenèse dans des adipocytes humains en culture.

## Revendications

1. Utilisation cosmétique d'une composition comprenant au moins une oxazoline, à titre de principe actif, comme amincissant.

2. Utilisation cosmétique d'une composition comprenant au moins une oxazoline, à titre de principe actif, pour prévenir et/ou traiter la cellulite.

3. Utilisation cosmétique selon l'une quelconque des revendications 1 ou 2 **caractérisée en ce que** ladite oxazoline répond aux formules générales suivantes: 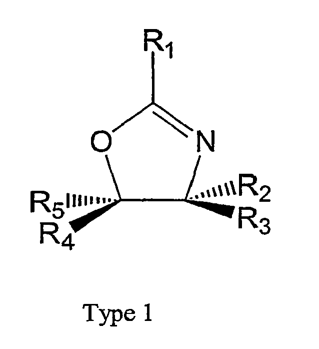 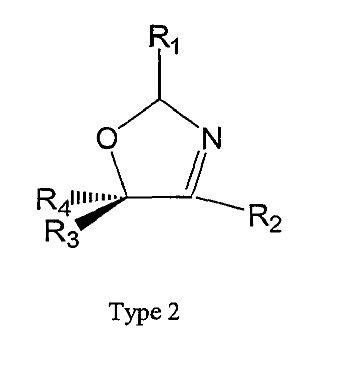 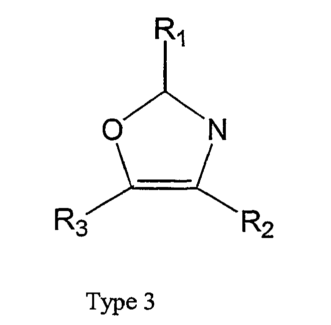 dans laquelle R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) ;
R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆).

4. Utilisation cosmétique selon l'une quelconque des revendications précédentes, **caractérisée en ce que** ladite oxazoline est une oxazoline de type 1 sélectionnée dans le groupe composé de la 2-undécyl-4-hydroxyméthyl-4-méthyl-1,3-oxazoline, de la 2-undécyl-4,4-diméthyl-1,3-oxazoline, de la (E)-4,4-diméthyl-2-heptadéc-8-ényl-1,3-oxazoline, de la 4-hydroxyméthyl-4-méthyl-2-heptadécyl-1,3-oxazoline, la (E)-4-hydroxyméthyl-4-mëthyl-2-heptadéc-8-ényl-1,3-oxazoline, la 2-undécyl-4-éthyl-4-hydroxyméthyl-1,3-oxazoline.

5. Utilisation cosmétique selon l'une quelconque des revendications précédentes, **caractérisée en ce que** ladite oxazoline est la 2-undécyl-4,4-diméthyl-1,3-oxazoline, appelée OX100, de formule:

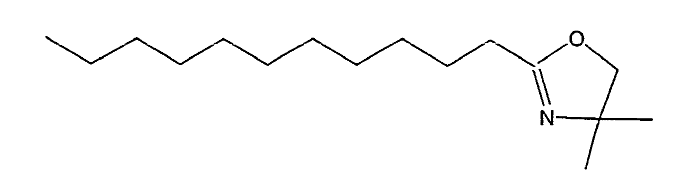

6. Utilisation cosmétique selon l'une quelconque des revendications précédentes, **caractérisée en ce que** la composition comprend entre 0,01 et 10% en poids d'oxazoline, avantageusement entre 0,01 et 3% en poids d'oxazoline, par rapport au poids total de la composition et un milieu cosmétiquement acceptable.

7. Utilisation cosmétique selon l'une quelconque des revendications précédentes, **caractérisée en ce que** l'on applique, de façon simultanée, séparée ou étalée dans le temps, une ou plusieurs oxazolines ainsi que un ou plusieurs actifs amincissants de type lipolytique et/ou un ou plusieurs actifs amincissants de type liporéducteur.

8. Utilisation cosmétique selon l'une quelconque des revendications précédentes, **caractérisée en ce que** l'on applique en outre de façon simultanée, séparée ou étalée dans le temps, un ou plusieurs actifs désinfiltrants et/ou veinotoniques.

9. Utilisation cosmétique selon l'une quelconque des revendications précédentes, **caractérisée en ce que** la composition est à application topique.

## Claims

1. Cosmetic use of a composition containing at least one zoline, serving as an active substance, as a slimming product.

2. Cosmetic use of a composition containing at least one zoline, serving as an active substance, for preventing and/or treating cellulite.

3. Cosmetic use according to either one of Claims 1 and 2, **characterized in that** said oxazoline corresponds to the general formulae below: 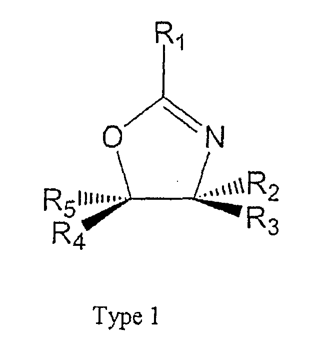 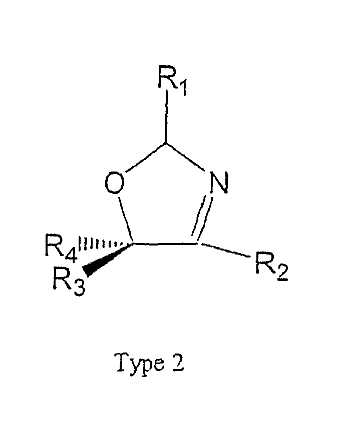 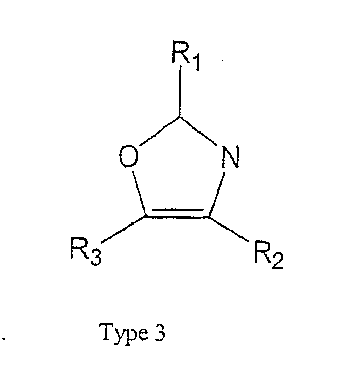 in which R₁ represents a linear or branched, saturated or unsaturated C₁-C₄₀ alkyl group optionally comprising one or more ethylenic unsaturation(s) and also one or more substituent(s) chosen from the group formed by hydroxyl (OH) and C₁-C₆ alkoxy (OC₁-C₆) radicals; R₂, R₃, R₄ and R₅ represent, independently, a hydrogen atom, a hydroxyl radical, or a linear or branched, saturated or unsaturated C₁-C₃₀ alkyl group optionally comprising one or more ethylenic unsaturations and also one or more substituent(s) chosen from the group formed by hydroxyl (OH), C₁-C₆ alkoxy (OC₁-C₆) and C₁-C₆ alkoxy carbonyl (COOC₁-C₆) radicals.

4. Cosmetic use according to any one of preceding claims, **characterized in that** said oxazoline is a type 1 oxazoline selected from the group composed of 2-undecyl-4-hydroxymethyl-4-methyl-1,3-oxazoline, 2-undecyl-4,4-dimethyl-1,3-oxazoline, (E)-4,4-dimethyl-2-heptadec-8-enyl-1,3-oxazoline, 4-hydroxymethyl-4-methyl-2-heptadecyl-1,3-oxazoline, (E)-4-hydroxymethyl-4-methyl-2-heptadec-8-enyl-1,3-oxazoline and 2-undecyl-4-ethyl-4-hydroxymethyl-1,3-oxazoline.

5. Cosmetic use according to any one of the preceding claims, **characterized in that** said oxazoline is 2-undecyl-4,4-dimethyl-1,3-oxazoline, called OX100, of formula:

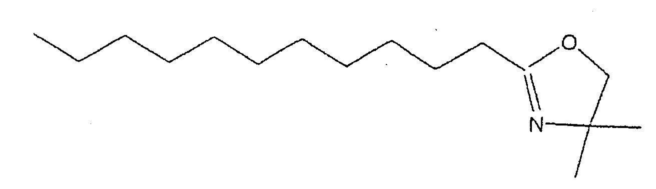

6. Cosmetic use according to any one of the preceding claims, **characterized in that** the composition comprises between 0.01 and 10% by weight of oxazoline, advantageously between 0.01 and 3% by weight of oxazoline, relative to the total weight of the composition, and a cosmetically acceptable medium.

7. Cosmetic use according to any one of the preceding claims, **characterized in that** one or more oxazolines and also one or more slimming active agents of lipolytic type and/or one or more slimming active agents of liporeducing type are applied simultaneously, separately or spread out over time.

8. Cosmetic use according to any one of the preceding claims, **characterized in that** one or more anti-infiltration and/or venotonic active agents are also applied simultaneously, separately or spread out over time.

9. Cosmetic use according to any one of the preceding claims, **characterized in that** the composition is for topical application.

## Patentansprüche

1. Kosmetische Verwendung einer Zusammensetzung, die wenigstens ein Oxazolin als Wirkstoff enthält, als schlanker machendes Mittel.

2. Kosmetische Verwendung einer Zusammensetzung, die wenigstens ein Oxazolin als Wirkstoff enthält, um Cellulitis zu verhüten und/oder zu behandeln.

3. Kosmetische Verwendung nach einem der Ansprüche 1 oder 2, **dadurch gekennzeichnet, dass** das Oxazolin den folgenden allgemeinen Formeln entspricht: 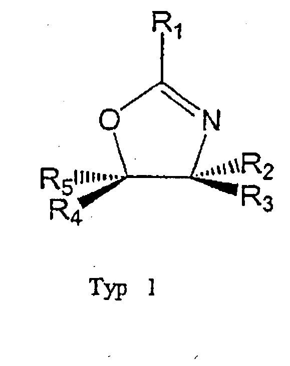 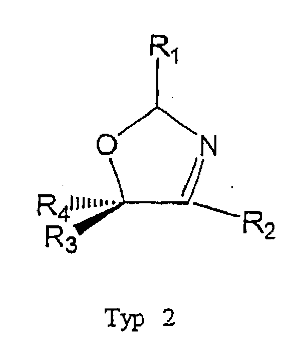 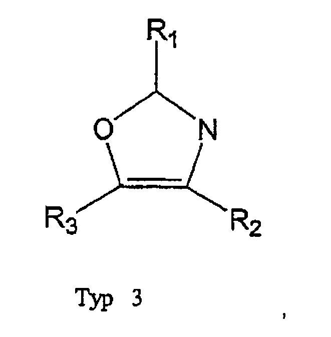 in welcher R₁ für eine lineare oder verzweigte, gesättigte oder ungesättigte C₁-C₄₀-Alkylgruppe steht, welche gegebenenfalls eine oder mehrere ethylenische Ungesättigtheit(en) wie auch einen oder mehrere Substituent(en), ausgewählt in der Gruppe, welche aus dem Hydroxyrest (OH) und den C₁-C₆-Alkoxyresten (OC₁-C₆) besteht, umfasst;
R₂, R₃, R₄ und R₅ unabhängig für ein Wasserstoffatom, einen Hydroxyrest oder eine lineare oder verzweigte, gesättigte oder ungesättigte C₁-C₃₀-Alkylgruppe, welche gegebenenfalls eine oder mehrere ethylenische Ungesättigtheiten wie auch einen oder mehrere Substituent(en), ausgewählt in der Gruppe, welche aus dem Hydroxyrest (OH), den C₁-C₆-Alkoxyresten (OC₁-C₆) und den C₁-C₆-Alkoxycarbonylresten (COOC₁-C₆) besteht, umfasst, stehen.

4. Kosmetische Verwendung nach einem der vorangegangenen Ansprüche, **dadurch gekennzeichnet, dass** das Oxazolin ein Oxazolin vom Typ 1 ist, welches in der aus 2-Undecyl-4-hydroxymethyl-4-methyl-1,3-oxazolin, 2-Undecyl-4,4-dimethyl-1,3-oxazolin, (E)-4,4-Dimethyl-2-heptadec-8-enyl-1,3-oxazolin, 4-Hydroxymethyl-4-methyl-2-heptadecyl-1,3-oxazolin, (E)-4-Hydroxymethyl-4-methyl-2-heptadec-8-enyl-1,3-oxazolin, 2-Undecyl-4-ethyl-4-hydroxymethyl-1,3-oxazolin bestehenden Gruppe ausgewählt wird.

5. Kosmetische Verwendung nach einem der vorangegangenen Ansprüche, **dadurch gekennzeichnet, dass** das Oxazolin 2-Undecyl-4,4-dimethyl-1,3-oxazolin, welches als OX100 bezeichnet wird, mit der Formel: 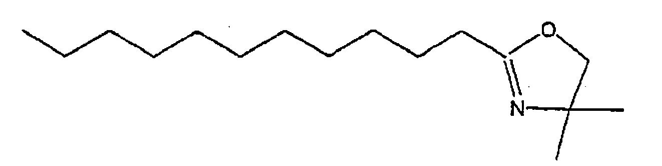 ist.

6. Kosmetische Verwendung nach einem der vorangegangenen Ansprüche, **dadurch gekennzeichnet, dass** die Zusammensetzung zwischen 0,01 und 10 Gew.-% Oxazolin, vorteilhafterweise zwischen 0,01 und 3 Gew.-% Oxazolin bezogen auf das Gesamtgewicht der Zusammensetzung und ein kosmetisch annehmbares Medium umfasst.

7. Kosmetische Verwendung nach einem der vorangegangenen Ansprüche, **dadurch gekennzeichnet, dass** man gleichzeitig, getrennt oder zeitlich gestaffelt ein oder mehrere Oxazoline wie auch einen oder mehrere schlanker machende Wirkstoffe vom lipolytischen Typ und/oder einen oder mehrere schlanker machende Wirkstoffe vom fettreduzierenden Typ anwendet.

8. Kosmetische Verwendung nach einem der vorangegangenen Ansprüche, **dadurch gekennzeichnet, dass** man außerdem gleichzeitig, getrennt oder zeitlich gestaffelt einen oder mehrere entschlackende und/oder venotonische Wirkstoffe anwendet.

9. Kosmetische Verwendung nach einem der vorangegangenen Ansprüche, **dadurch gekennzeichnet, dass** die Zusammensetzung für eine topische Anwendung bestimmt ist.
